**Europäisches Patentamt**

**European Patent Office**

**Office européen des brevets**

(19)

(11) Numéro de publication : **0 489 884 B1**

(12) **FASCICULE DE BREVET EUROPEEN**

(45) Date de publication du fascicule du brevet :
02.03.94 Bulletin 94/09

(51) Int. Cl.⁵ : **A23C 9/14,** A23C 9/146, B01D 15/08, C12N 5/00

(21) Numéro de dépôt : **91912035.2**

(22) Date de dépôt : **24.06.91**

(86) Numéro de dépôt international :
**PCT/FR91/00502**

(87) Numéro de publication internationale :
**WO 92/00014 09.01.92 Gazette 92/02**

(54) PROCEDE DE TRAITEMENT DU COLOSTRUM PAR CHROMATOGRAPHIE D'ADSORPTION SUR HYDROXYAPATITE, FRACTION ACTIVE DE COLOSTRUM OBTENUE ET MILIEU CELLULAIRE CONTENANT LADITE FRACTION ACTIVE.

(30) Priorité : **28.06.90 FR 9008573**

(43) Date de publication de la demande :
**17.06.92 Bulletin 92/25**

(45) Mention de la délivrance du brevet :
**02.03.94 Bulletin 94/09**

(84) Etats contractants désignés :
**AT BE CH DE DK ES GB GR IT LI LU NL SE**

(56) Documents cités :
**EP-A- 0 085 005**
**FR-A- 2 487 642**
**US-A- 4 440 860**
**Patent Abstracts of Japan, vol. 4, No. 30 (C-2)[512], 15 March 1980**

(72) Inventeur : **QUINQUE, Bernard**
**2015, route Nationale**
**F-62140 Marconnelle (FR)**
Inventeur : **LESUR, Hélène**
**8, rue Bodelot**
**F-62460 Divion (FR)**
Inventeur : **SPIK, Geneviève**
**39, résidence du Moulin, Rue de la Pilaterie**
**F-59700 Marcq-en-Baroeul (FR)**
Inventeur : **MONTREUIL, Jean**
**145, rue Boucly**
**F-59650 Villeneuve-d'Ascq (FR)**
Inventeur : **THOMAS, Daniel**
**33, allée de l'Etang, Domaine de Rimderlieu**
**F-60150 Villers-sur-Coudun (FR)**

(74) Mandataire : **Hennion, Jean-Claude et al**
**Cabinet Beau de Loménie, 37, rue du Vieux Faubourg**
**F-59800 Lille (FR)**

(73) Titulaire : **CLAR (SARL)**
**Domaine de Radinghem**
**F-62310 Fruges (FR)**

EP 0 489 884 B1

## Description

DOMAINE TECHNIQUE

La presente invention concerne le traitement du colostrum , notamment en vue d'obtenir une fraction de colostrum particulièrement riche en facteurs de croissance et dépourvue de protéases. Elle concerne le procédé de traitement proprement dit ainsi que la fraction de colostrum specialement obtenue au cours du traitement. Elle concerne également l'utilisation dans des milieux de culture d'une fraction de colostrum riche en facteurs de croissance et pauvre en protéases, dénommée fraction active

TECHNIQUE ANTERIEURE

Dans le domaine de la culture cellulaire, le sérum est l'additif le plus utilisé pour stimuler la prolifération cellulaire. Ce liquide biologique renferme les facteurs nécessaires à la nutrition, à la croissance et au développement des cellules de l'organisme. Son action est imparfaitement connue et sa composition très variable d'un lot à l'autre. De plus c'est un produit soumis à des fluctuations d'approvisionnement et coûteux. On recherche donc des substituts du sérum, aptes au développement de milieux de culture cellulaire parfaitement définis

On a proposé de remplacer le sérum par des produits laitiers, tels que des fractions de lait ou de lactosérum de vache, par exemple dans le document FR.A.2.586 699 des fractions de lait formées de constituants ayant un poids moléculaire d'au moins environ 27.000 et dépourvues de lipides insolubles du type de ceux éliminables par ultracentrifugation. De telles fractions sont obtenues en soumettant une phase limpide de lait renfermant les protéines solubles du lait, obtenue par ultracentrifugation et séparation, à une ultrafiltration avec une membrane à seuil de coupure de 50 à 27.000 daltons environ.

Parmi les produits laitiers, on a proposé de remplacer le sérum par le colostrum, qui est le liquide secrété par la glande mammaire dans les quelques jours qui suivent une naissance.Dans le document US.A.4,440,860 il est décrit une fraction du colostrum comme facteur de croissance particulièrement actif, cette fraction a un poids moléculaire inférieur à 20.000 et un point isoélectrique compris entre 4,4 et 4,8. Cette fraction est obtenue par un écrémage , suivi d'une mise en contact du colostrum écrémé avec de l'acide acétique pH 4,3 pendant 2 heures et centrifugation pour éliminer le précipité, puis par purification basée sur le point isoélectrique , filtration sur un gel et éventuellement concentration par électrophorèse.

Les procédés précités concernent une sélection de composants basée sur le poids moléculaire.

D'autres procédés visent à isoler le facteur de croissance principal du colostrum. De tels procédés sont décrits par Yuen Shing et Michael Klagsbrum dans l'article intitulé "Purification and Characterization of a Bovine Colostrum-Derived Growth Factor" paru dans Molecular Endocrinology 87/0335 et par Yuen Shing , Susan Davidson et Michael Klagsbrum dans l'article intitulé "Purification of Polypeptide Growth Factors from Milk" paru dans Methods in Enzymology - 1987 - volume 146 - pages 42-48 .Selon ces auteurs , le facteur de croissance principal est le BCGF (Bovine Colostrum Growth Factor) dans le cas du colostrum de vache, ayant un poids moléculaire d'environ 30.000, et le PDGF (human platelet-derived growth factor) dans le cas du colostrum humain, ayant un poids moléculaire d'environ 20.000. Pour isoler le facteur de croissance principal du colostrum bovin, les procédés en question comportent une succession d'opérations : écrémage, précipitation par voie acide et par ébullition, chromatographie d'échange d'ions, chromatographie d'isoélectrofocalisation ou bien écrémage, filtration, chromatographie d'échange d'ions, chromatographie d'isoélectrofocalisation , cette opération étant suivie dans les deux cas par une séparation du type HPLC reverse phase . Dans ces procédés le rendement obtenu en facteurs de croissance est de l'ordre de 1%, c'est-à-dire qu'au cours des différentes opérations mises en oeuvre, 99% de l'activité en facteurs de croissances du colostrum initial ont été perdus.

EXPOSE DE L'INVENTION

Le but que s'est fixé le demandeur est différent de ceux poursuivis par les procédés précités. Il cherche dans un premier temps à obtenir une fraction du colostrum, qui soit enrichie en facteurs de croissance, mais sans qu'il y ait une particulière purification du facteur de croissance principal, et qui de plus soit pauvre en protéases.

Le demandeur a , en effet, constaté que la présence des protéases , qui sont des enzymes hydrolysant les protides empêchait une bonne conservation dans le temps des fractions de colostrum enrichies en facteurs de croissance et de plus entraînait une baisse progressive de l'activité desdites fractions.

Le but visé est parfaitement atteint par le procédé de l'invention. Ce procédé de traitement du colostrum comprend de manière connue au moins une opération de chromatographie d'échange de cations. Selon l'invention, on soumet la partie du colostrum qui a été déplacée par élution de l'échangeur de cations à une chromatographie d'adsorption sur hydroxyapatite et on récupère par élution la fraction retenue sur l'hydroxyapatite.

On sait que l'hydroxyapatite est un phosphate tricalcique cristallisé et qu'il est utilisé comme support d'adsorption pour le fractionnement des protéines.

C'est le mérite de l'invention que d'avoir expérimenté et vérifié que l'utilisation de l'hydroxyapatite permettait non seulement de parfaire l'enrichissement en facteurs de croissance mais aussi d'éliminer les protéases de la fraction ainsi enrichie.

Plus particulièrement on récupère la fraction retenue sur l'hydroxyapatite par lavage par un tampon phosphate à une concentration au plus de 500mM.

Par exemple, on procède à un premier lavage de l'hydroxyapatite par un tampon phosphate pH 6,8 environ à une concentration au plus de 50mM puis à un second lavage par un tampon phosphate pH 6,8 environ à une concentration de l'ordre de 100mM. C'est ce second lavage qui constitue la fraction du colostrum enrichie en facteurs de croissance et pauvre en protéases. De préférence cette fraction est dessalée , concentrée et congelée ou séchée par exemple par lyophilisation ou par atomisation.

Avantageusement , l'échangeur de cation étant à fonctions sulfoniques, on obtient la partie du colostrum déplacée dudit échangeur, après un premier lavage de l'échangeur avec une solution au plus 0,1M d'acétate de sodium , par lavage de l'échangeur par une solution environ 0,5M d'acétate de sodium.

Avantageusement on ne procède préalablement à la chromatographie d'échange de cations, qu'à une seule opération qui est un écrémage portant la teneur en matière grasse du colostrum à 2g/l au plus.

Selon le mode préféré de réalisation du procédé de l'invention , on procède sur le colostrum aux opérations successives suivantes :

a. écrémage, portant la teneur en matière grasse à 2g/l au plus

b. passage du colostrum écrémé sur un échangeur à fonctions sulfoniques

c. premier lavage de l'échangeur avec une solution au plus 0,1M d'acétate de sodium

d. second lavage de l'échangeur avec une solution d'acétate de sodium 0,5M environ

e. passage de la partie correspondant au second lavage (d), dessalée et concentrée, sur de l'hydroxyapatite équilibrée à 10mM environ

f. premier lavage de l'hydroxyapatite par un tampon phosphate pH6,8 environ à 50mM au plus

g. second lavage de l'hydroxyapatite par un tampon phosphate pH6,8 environ à 100mM environ

h. dessalage, concentration et congélation ou séchage de la partie correspondant au second lavage (g)

La fraction de colostrum ainsi obtenue , dite fraction active, comporte plus de 50% de l'ensemble des facteurs de croissance du colostrum initial et est pratiquement exempte de protéases.

C'est un autre objet de l'invention que de protéger des milieux de culture cellulaire comportant de la fraction active comme additif pour stimuler la prolifération cellulaire. Les types cellulaires concernés sont des cellules d'eucaryotes, entre autres fibroblastes, hybridomes, cellules épithéliales, cellules endothéliales, hépatocytes, lymphocytes, macrophages, adipocytes, cellules hématopoïétiques, cellules granulo-monocytaires, cellules cardiaques, cellules nerveuses, cellules musculaires, cellules cutanées, kératinocytes, mélanocytes, chondrocytes, cellules d'insecte.

Un milieu de culture cellulaire particulièrement intéressant comporte du sérum de veau foetal et de la fraction active. L'action combinée de la fraction active et du sérum permet de diminuer de façon très importante la quantité de sérum mise en oeuvre pour une croissance cellulaire équivalente. La fraction active peut trouver son utilité dans d'autres secteurs, notamment pharmaceutique ou cosmétologique.

## MEILLEURE MANIERE DE REALISER L'INVENTION

D'autres avantages et caractéristiques de l'invention ressortiront plus clairement de la description qui va être faite d'un exemple de traitement du colostrum sur hydroxyapatite.

Le terme colostrum désigne le mélange de sécrétions lactées et de constituants du sérum sanguin, obtenu dans les 7 jours suivant la mise-bas (post-partum) . Il provient de la vache ou d'autres mammifères.

On part d'un colostrum de vache , auquel on fait subir un écrémage par centrifugation à 7900 tours par minute de manière à éliminer la plupart des matières grasses . Sans autre traitement préalable, un litre de colostrum écrémé , ayant une teneur en matières grasses inférieure à 2g/l, est mis en contact avec 15g d'une résine échangeuse de cations, à fonctions sulfoniques, commercialisée par la société PHARMACIA sous la référence SP-SEPHADEX® C-50. Le pH est de 6,3 . La mise en contact a lieu sous agitation pendant une durée d'au moins 4 heures et à 4°C.

La partie du colostrum écrémé restant dans le bain contient tous les composants qui n'ont pas été retenus par les fonctions sulfoniques de la résine. Cette partie du colostrum est pratiquement exempte de facteurs de croissance ; cependant elle présente un intérêt du fait qu'elle est riche entre autres composants en immunoglobulines colostrales , en sucres, en enzymes et en protéose-peptones. Elle pourra être utilisée telle quelle ou après séparation desdits composants.

Après élimination du bain, la résine est d'abord lavée à l'aide d'une solution 0,1 molaire d'acétate de sodium. Ce premier lavage a pour but d'enlever de la surface de la résine les composants qui ne sont pas à proprement parler retenus par échange ionique sur les fonctions sulfoniques.

On procède ensuite à un deuxième lavage de la résine à l'aide d'une solution 0,5 molaire d'acétate de sodium. Ce lavage a pour effet de déplacer une partie des composants du colostrum qui étaient retenus par échange ionique sur les fonctions sulfoniques de la résine. Ce déplacement est fonction de différents paramètres tels l'affinité respective des composants pour les fonctions sulfoniques , la cinétique d'échange , l'effet stérique.

On procède enfin à un troisième lavage de la résine à l'aide d'une solution 1,0 molaire d'acétate de sodium.

Les deux solutions de lavage à 0,5 et 1,0M d'acétate de sodium sont respectivement dessalées par ultrafiltration, dialyse, électrodialyse, ou tamisage moléculaire , puis concentrées par ultrafiltration avec un seuil de coupure de 5.000 de masse moléculaire.

Une analyse comparative du colostrum écrémé initial, et des deux fractions correspondant l'une au lavage 0,5M et l'autre au lavage 1,0M fait apparaître d'une part que plus de 80 % des facteurs de croissance contenus dans le colostrum écrémé se trouvent dans les deux fractions, et d'autre part que la fraction correspondant au lavage 0,5M, contient 73,2% des facteurs de croissance contenus dans le colostrum écrémé. Cette fraction contient également entre autres composants de la lactoperoxydase, de la lactotransferrine , du lyzozyme et des protéases.

Le tableau I ci-dessous donne les résultats comparés entre le colostrum écrémé (A) et la fraction éluée à 0,5M.

## Tableau I

|  | (A) colostrum écrémé | (B) fraction éluée à 0,5M |
|---|---|---|
| protéines totales (mg) | 130.000 | 1.620 |
| activité totale (U) | 130.000 | 95.256 |
| activité spécifique (U/mg) | 1 | 58,8 |
| rendement d'activité (%) | 100 | 73,2 |

La teneur en protéines a été dosée par la technique LOWRY, modifiée par PETERSON. L'unité U de facteur de croissance, encore appelée activité mitogène, est égale à la quantité de protéines nécessaires pour provoquer une incorporation de méthyl-thymidine tritiée égale à la moitié de l'incorporation maximale.

Cette fraction (B) est dessalée et concentrée, puis injectée sur une colonne d'hydroxyapatite préalablement équilibrée dans un tampon phosphate pH6,8 à 10mM. La quantité d'hydroxyapatite , qui a la forme d'un gel, est déterminée en fonction de la quantité de protéines présentes dans la fraction, de façon à ce qu'il y ait au plus 40mg de protéines par millilitre de gel d'hydroxyapatite.

On élue ensuite la colonne d'hydroxyapatite successivement par trois solutions tampons phosphates pH 6,8 à des concentrations de plus en plus concentrées , respectivement 50mM pour le premier, 100mM pour le deuxième et 500mM pour le troisième.

Chacune des fractions obtenues est dessalée , concentrée et lyophilisée. Les fractions 50 et 500mM renferment quasiment la totalité des protéases. Seule la fraction 100mM est quasiment exempte de protéases.

La deuxième fraction, correspondant à l'élution par la solution à 100mM , dite fraction active, est la seule enrichie en facteurs de croissance.

Dans le tableau II ci-dessous , on a complété les résultats donnés dans le tableau I à l'aide des informations relatives à ladite fraction active (C).

## Tableau II

| | (A) colostrum écrémé | (B) échange d'ions élution à 0,5M | (C) hydroxyapatite élution à 0,1M |
|---|---|---|---|
| Protéines totales | 130.000 mg | 1.620 mg | 648 mg |
| Activité totale | 130.000 U | 95.256 U | 76.205 U |
| Activité spécifique | 1 U/mg | 58,8U/mg | 117,6 U/mg |
| Rendement | 100 % | 73,2 % | 59 % |

Par rapport au colostrum écrémé (A), la fraction active (C) se distingue par une très forte activité spécifique.

Une comparaison a été faite entre la fraction active et le sérum de veau foetal qui est l'additif le plus répandu pour stimuler la prolifération cellulaire. Pour cela on a utilisé un test d'incorporation de thymidine tritiée pour détecter les activités mitogènes respectives sur la souche de fibroblastes CCL39. Il ressort de cette comparaison que l'activité spécifique de la fraction active (117,6U/mg de protéines)est nettement plus importante que celle du sérum de veau foetal (15 U/mg de protéines). Ceci est un réel avantage pour la fraction active dans la mesure où il est souvent nécessaire d'éliminer ultérieurement du milieu de culture les protéines qui ont été introduites avec l'additif ; dans le cas de la fraction active la quantité de protéines à éliminer est faible.

La fraction active est utile en remplacement ou en combinaison avec le sérum de veau foetal, dans les milieux de culture cellulaire, par exemple dans un milieu MEM (Milieu Essentiel Minimum de Eagle) additionné de 2,2g/l de bicarbonate de soude, de 1% en volume de glutamine, 0,4 % en volume de pénicilline, de 0,4% de streptomycine, de 0,5% en volume de fungizone.

La fraction active a été utilisée comme additif de croissance dans un milieu de culture pour des fibroblastes CCL 39 en complément du sérum de veau foetal. Les plaques de culture, préalablement traitées ou non à la fibronectine, ont été ensemencées à raison de 160 000 cellules et 2 ml de milieu par puits, ledit milieu comprenant une certaine quantité de fraction active.

Par exemple l'addition de 1 % en volume de sérum de veau foetal et de 1 % en volume de la fraction active (contenant 1 g/l de protéines) dans un milieu de culture de fibroblastes permet d'obtenir une croissance cellulaire correspondant à celle obtenue avec environ 9 % en volume de sérum de veau foetal dans le même milieu.

L'invention n'est pas limitée au mode de réalisation qui a été décrit à titre d'exemple. Les conditions du traitement revendiquées ne sont pas limitées à ce qui a été décrit, d'autres types d'échangeurs de cations, autres que la résine, peuvent être mis en oeuvre, de même que des échangeurs ayant une autre fonction que la fonction sulfonique ; le déplacement de la partie du colostrum retenue sur l'échangeur de cation peut être obtenu par toute voie habituelle de régénération dudit échangeur, et le fonctionnement approprié sera déterminé au cas par cas.

## Revendications

1. Procédé de traitement du colostrum comprenant au moins une opération de chromatographie d'échange de cations caractérisé en ce qu'on soumet la partie du colostrum , qui a été déplacée par élution de l'échangeur de cations, à une chromatographie d'adsorption sur hydroxyapatite et on récupère par élution la fraction retenue sur l'hydroxyapatite.

2. Procédé selon la revendication 1 caractérisé en ce qu'on récupère la fraction retenue sur l'hydroxyapatite par lavage par un tampon phosphate à une concentration au plus de 500mM.

3. Procédé selon la revendication 2 caractérisé en ce qu'on procède à un premier lavage de l'hydroxyapatite par un tampon phosphate à une concentration au plus de 50mM puis à un second lavage par un tampon phosphate à une concentration de l'ordre de 100mM , la partie récupérée de ce second lavage correspondant à la fraction active enrichie en facteurs de croissance et pauvre en protéases.

4. Procédé selon la revendication 3 caractérisé en ce que la fraction active est dessalée, concentrée et congelée ou séchée.

5. Procédé selon l'une des revendications 1 à 4 caractérisé en ce que , l'échangeur de cations étant à fonctions sulfoniques, on obtient la partie du colostrum déplacée dudit échangeur, après un premier lavage de l'échangeur avec une solution au plus 0,1M d'acétate de sodium, par lavage de l'échangeur par une solution de l'ordre de 0,5M d'acétate de sodium.

6. Procédé selon la revendication 1 caractérisé en ce que préalablement à la chromatographie d'échange de cations, on ne procède sur le colostrum qu'à une seule opération qui est un écrémage , portant la teneur en matière grasse du colostrum à 2g/l au plus.

7. Procédé selon la revendication 6 caractérisé en ce que on a procédé sur le colostrum aux opérations successives suivantes :
   a. écrémage, portant la teneur en matière grasse à 2g/l au plus
   b. passage du colostrum écrémé sur un échangeur à fonctions sulfoniques
   c. premier lavage de l'échangeur avec une solution salée au plus 0,1M d'acétate de sodium
   d. second lavage de l'échangeur avec une solution d'acétate de sodium 0,5M environ
   e. passage de la partie correspondant au second lavage (d) dessalée et concentrée, sur de l'hydroxyapatite équilibrée à environ 10mM en tampon phosphate
   f. premier lavage de l'hydroxyapatite par un tampon phosphate pH de l'ordre de 6,8 à 50mM au plus
   g. second lavage de l'hydroxyapatite par un tampon phosphate pH de l'ordre de 6,8 à environ 100mM
   h. dessalage, concentration et congélation ou séchage de la partie résultant du second lavage (g) et correspondant à la fraction active

8. Fraction active de colostrum obtenue par le procédé de l'une des revendications 3 ou 7 caractérisée en ce qu'elle comporte plus de 50% des facteurs de croissance du colostrum initial et qu'elle est pratiquement exempte de protéases.

9. Milieu de culture cellulaire caractérisé en ce qu'il comporte de la fraction active selon la revendication 8 comme additif pour stimuler la prolifération cellulaire.

10. Milieu selon la revendication 9 caractérisé en ce qu'il concerne la culture de cellules d'eucaryotes, notamment fibroblastes,hybridomes, cellules epithéliales, cellules endothéliales, hépatocytes, lymphocytes, macrophages, adipocytes, cellules hématopoïétiques, cellules granulo-monocytaires, cellules cardiaques, cellules nerveuses, cellules musculaires, cellules cutanées, kératinocytes, mélanocytes, chondrocytes, cellules d'insecte.


## Patentansprüche

1. Verfahren zur Behandlung von Kolostrum, das zumindest einen Chromatographieschritt mit Kationenaustausch umfaßt, dadurch gekennzeichnet, daß man den Teil des Kolostrums, der durch die Elution vom Kationenaustauscher gewonnen wurde, einer Adsorptionschromatographie an Hydroxylapatit unterwirft und die am Hydroxylapatit zurückgehaltene Fraktion durch Elution gewinnt.

2. Verfahren nach Anspruch 1, dadurch gekennzeichnet, daß man die am Hydroxylapatit zurückgehaltene Fraktion durch Waschen mit einem Phosphatpuffer einer Konzentration von höchstens 500 mM zurückgewinnt.

3. Verfahren nach Anspruch 2, dadurch gekennzeichnet, daß man so verfährt, daß auf ein erstes Waschen des Hydroxylapatits mit einem Phosphatpuffer einer Konzentration von höchstens 50 mM ein zweites Waschen mit einem Phosphatpuffer einer Konzentration in der Größenordnung von 100 mM folgt, wobei der bei diesem zweiten Waschschritt gewonnene Teil der aktiven Fraktion entspricht, die an Wachstumsfaktoren angereichert und arm an Proteasen ist.

4. Verfahren nach Anspruch 3, dadurch gekennzeichnet, daß die aktive Fraktion entsalzt, konzentriert und eingefroren oder getrocknet wird.

5. Verfahren nach einem der Ansprüche 1 bis 4, dadurch gekennzeichnet, daß ein Kationenaustauscher mit Sulfonsäuregruppen verwendet wird und den vom Kationenaustauscher gewonnenen Teil des Kolostrums erhält, indem nach einem ersten Waschen des Kationenaustauschers mit einer höchstens 0,1 M Natriumacetatlösung ein Waschen des Kationenaustauschers mit einer Lösung folgt, die Natriumacetat in einer Konzentration in einer Größenordnung von 0,5 M enthält.

6. Verfahren nach Anspruch 1, dadurch gekennzeichnet, daß man vor der Kationenaustausch-Chromatographie nur einen Verfahrensschritt mit dem Kolostrum durchführt, der eine Entrahmung darstellt, durch die der Fettgehalt des Kolostrums auf höchstens 2 g/l gesenkt wird.

7. Verfahren nach Anspruch 6, dadurch gekennzeichnet, daß das Kolostrum nacheinander folgenden Verfahrensschritten unterzogen wird:
   a. Entrahmung, durch die der Fettgehalt auf höchstens 2 g/l gesenkt wird,
   b. Aufgeben des entrahmten Kolostrums auf einen Kationenaustauscher mit Sulfonsäuregruppen,
   c. erstes Waschen des Kationenaustauschers mit einer höchstens 0,1 M Natriumacetatlösung,
   d. zweites Waschen des Kationenaustauschers mit einer ungefähr 0,5 M Natriumacetatlösung,
   e. Aufgeben des entsalzten und konzentrierten Teils vom zweiten Waschen (d) auf den Hydroxylapatit, der mit ungefähr 10 mM Phosphatpuffer äquilibriert wurde,
   f. erstes Waschen des Hydroxylapatits mit einem Phosphatpuffer von etwa pH 6,8 und einer Konzentration von höchstens 50 mM,
   g. zweites Waschen des Hydroxylapatits mit einem Phosphatpuffer mit einem pH-Wert von etwa 6,8 und einer Konzentration von ungefähr 100 mM,
   h. Entsalzen, Konzentrieren und Einfrieren des aus dem zweiten Waschen (g) erhaltenen Teils, welcher der aktiven Fraktion entspricht.

8. Aktive Fraktion aus Kolostrum, die nach dem Verfahren nach Anspruch 3 oder 7 erhalten wurde, dadurch gekennzeichnet, daß sie mehr als 50 % der Wachstumsfaktoren des ursprünglichen Kolostrums enthält und weitgehend von Proteasen befreit ist.

9. Zellkulturmedium, dadurch gekennzeichnet, daß es die aktive Fraktion nach Anspruch 8 als Zusatz enthält, um die Zellproliferation anzuregen.

10. Zellkulturmedium nach Anspruch 9, dadurch gekennzeichnet, daß es für Zellkulturen von Eukaryoten, insbesondere Fibroblasten, Hybridomzellen, Epithelzellen, Endothelzellen, Hepatocyten, Lymphocyten, Makrophagen, Adipocyten, blutbildenden Zellen, Granulo-monocyten, Herzmuskelzellen, Nervenzellen, Muskelzellen, Hautzellen, Keratinocyten, Melanocyten, Chondrocyten und Insektenzellen vorgesehen ist.

**Claims**

1. Method for treating colostrum comprising at least one operation of cation exchange chromatography, characterized in that the part of colostrum which was shifted by elution of the cation exchanger, is subjected to an adsorption chromatography on hydroxyapatite and the fraction retained on the hydroxyapatite is recovered by elution.

2. Method according to claim 1, characterized in that the fraction retained on the hydroxyapatite is recovered by washing with a phosphate buffer at a concentration of 500mM maximum.

3. Method according to claim 2, characterized in that it involves a first wash of the hydroxyapatite with a phosphate buffer at a concentration of 50mM maximum followed by a second wash with a phosphate buffer at a concentration of about 100mM, the part recovered from this second wash corresponding to the active fraction enriched with growth factors and poor in proteases.

4. Method according to claim 3, characterized in that the active fraction is desalted, concentrated and frozen or dried.

5. Method according to one of claims 1 to 4, characterized in that the cation exchanger being with sulfonic functions, the colostrum part shifted from said exchanger, is obtained after a first wash of the exchanger with a 0.1M maximum sodium acetate solution, by washing of the exchanger with a sodium acetate solution of about 0.5M.

6. Method according to claim 1, characterized in that only one operation is carried out prior to the cation exchange chromatography, and that is a skimming operation to bring the fats content of the colostrum to 2g/l maximum.

7. Method according to claim 6, characterized in that the following sequence of operations is performed on the colostrum:
   a. skimming, to bring the fats content to 2g/l maximum
   b. passage of the skimmed colostrum on an exchanger having sulfonic functions
   c. first wash of the exchanger with a salted solution of 0.1M sodium acetate maximum
   d. second wash of the exchanger with a solution of about 0.5M of sodium acetate
   e. passage of the part corresponding to the second wash (d), desalted and concentrated, on hydroxyapatite balanced to about 10mM in phosphate buffer
   f. first wash of the hydroxyapatite with a phosphate buffer of pH about 6.8 at 50mM maximum
   g. second wash of the hydroxyapatite with a phosphate buffer of pH about 6.8 at about 100mM
   h. desalting, concentrating and freezing or drying of the part resulting from the second wash (g) and corresponding to the active fraction.

8. Active fraction of colostrum obtained by the method of one of claims 3 or 7, characterized in that it comprises more than 50% growth factors from the initial colostrum and it is virtually free of proteases.

9. Cell culture medium, characterized in that it comprises some active fraction according to claim 8, as additive for promoting cell proliferation.

10. Medium according to claim 9, characterized in that it is concerned with the culture of eukaryote cells, among which fibroblasts, hybridomae, epithelial cells, endothelial cells, hepatocytes, lymphocytes, macrophages, adipocytes, hematopoietic cells, granulomonocytic cells, heart cells, nervous cells, muscle cells, cutaneous cells, keratinocytes, melanocytes, chondrocytes, insect cells.